(19) Europäisches Patentamt
European Patent Office
Office européen des brevets

(11) Publication number: **0 070 128**
**B1**

(12) **EUROPEAN PATENT SPECIFICATION**

(45) Date of publication of patent specification: **04.06.86**

(51) Int. Cl.$^4$: **A 61 F 9/00**, A 61 H 33/04, A 61 M 7/00

(21) Application number: **82303479.8**

(22) Date of filing: **02.07.82**

(54) **Eye irrigating apparatus.**

(30) Priority: **15.07.81 US 283555**

(43) Date of publication of application:
**19.01.83 Bulletin 83/03**

(45) Publication of the grant of the patent:
**04.06.86 Bulletin 86/23**

(84) Designated Contracting States:
**BE DE FR GB IT NL**

(56) References cited:
**DE-C- 594 860**
**FR-A-1 213 031**
**US-A-2 078 180**
**US-A-2 366 424**
**US-A-2 702 540**
**US-A-2 908 476**
**US-A-4 131 115**

(73) Proprietor: **Ehrlich, Kenneth Bruce**
**101 Temple Avenue**
**Manahawkin New Jersey (US)**

(72) Inventor: **Ehrlich, Kenneth Bruce**
**101 Temple Avenue**
**Manahawkin New Jersey (US)**

(74) Representative: **Gilmour, David Cedric Franklyn et al**
**POTTS, KERR & CO. 15 Hamilton Square**
**Birkenhead Merseyside L41 6BR (GB)**

**EP 0 070 128 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to apparatus for enabling the irrigation of an eye in desirable manner.

German Patent Specification No. 594860 discloses a device for supplying drops to an eye and includes scissor like levers having arms for opening the eyelids and a pipette which is connected to a rubber tube which acts as a liquid reservoir and which enables drops to be supplied to an eye therebeneath by the squeezing thereof manually or by the action of closing the scissor lever ends remote from the eyelid opening arms. This device must be disposed with the pipette and tube reservoir in a vertical plane and only enables a limited supply of drops to be supplied to an eye which would be insufficient for certain irrigation purposes and can prove difficult to manipulate.

U.S. Patent Specification No. 4,131,115 discloses a self-operable eye-washing bottle of somewhat complex construction and wherein water is supplied in an upwardly directed jet under the action of a manually compressible air chamber and thus operation can prove difficult and the effect of irrigation cannot be readily inspected during irrigation and the pressure of liquid supplied cannot be adjusted for constant flow nor is the supply unlimited. Furthermore accuracy in directioning of the water jet is not readily achievable.

Hitherto no apparatus has been available which can be used by an individual to spread the eyelids and keep them open for a prolonged period of time, while at the same time providing an unlimited stream of irrigating fluid (not drops), delivered with proper selected pressure so as to not damage the eye, and directed into the eye to irrigate it with an irritating substance.

According to the present invention there is provided eye irrigation apparatus comprising a scissors-like mechanism comprising two blades each having a finger hole at one end and eyelid engaging means at the other end, and which blades are pivotably connected, characterised in that there are provided a tubular valve body disposed and supported between said blades, the valve body being adapted to receive fluid from a source via a tube and being positioned so that fluid flowing in a stream out of said valve body flows into and irrigates the eye when the apparatus is disposed in operative relation with the eye, and an adjustable valve means in said valve body for controlling the flow of fluid therethrough and settable over a range of settings to permit steady fluid flow therethrough at a selected velocity.

It will be appreciated that the irrigation apparatus of the invention enables an unlimited source of universally available irrigation fluid delivered at a safe (preadjusted) rate of pressure which permits immediate, maximum and most effective irrigation of the eye; eliminates or minimises the need for manual manipulation of the eyelids, therefore permitting more complete prolonged irrigation for optimum treatment, and is simple and economic to use.

Preferably the invention further contains the features defined in sub-claims 2 to 9.

Ways of carrying out the invention are described in detail below with reference to the accompanying drawings which illustrate an embodiment of the invention, in which:—

Fig. 1 is a perspective view of the eye irrigation apparatus;

Fig. 2 is a side elevational view of the apparatus of Fig. 1;

Fig. 3 is a plan view of the apparatus of Fig. 2;

Fig. 4 is a sectional view along the line 4—4 of Fig. 3;

Fig. 5 is a view, partly in section, through apparatus near its center;

Fig. 6 is a perspective view of a portion of the apparatus; and

Fig. 7 is a side view of a modification of a portion of the apparatus of the invention.

The drawings show eye irrigating apparatus 10 comprising a scissors-like device including first and second blades 20 and 30 with blade 20 having a finger hole 40 and with blade 30 having a finger hole 50 at one end. At the opposite end, each blade has a pad 60 of a soft material such as foam rubber having a surface which is not smooth, but is provided with small bumps or projections which permit frictional engagement of the pads 60 with the eyelids of a person.

The blades 20 and 30 are pivotably coupled together at about their centers about pins or posts 64. In one coupling arrangement, the outer surface of each blade 20, 30 carries a disk 33, and a U-shaped clamp 35 engages the two disks with a tight mechanical fit to hold the parts together. Other coupling arrangements may also be used. The blades 20 and 30 are shaped and disposed so that the ends of the blades work in opposite directions; that is when the finger hole portions 40 and 50 are together, the pads 60 are apart, and vice versa. This is done by having the blades not cross each other as in conventional scissors.

Each blade carries a raised stud 61 and 62 on its inner face, just rearward of the pivot points 64. The studs extend toward each other, but they are offset one above the other (Fig. 4) for a purpose to be described.

A tubular plastic valve body 70, through which the irrigating fluid may flow, is positioned between the blades 20 and 30 which are suitably shaped for this purpose. The valve body 70 is held in place, for example, by means of the pins or posts 64, about which the blades 20 and 30 are free to pivot or rotate with respect to each other.

A flexible plastics material tube 110 is disposed within the valve body, and a separate nozzle or tip 112 is secured to the front end of the flexible tube and is suitably secured or cemented to the front end of the valve body 70. The flexible tube 110 has its end remote from the tip 112 provided with means for connection to a fluid source 120, for example, a water faucet. The valve body 70 is provided with a threaded hub 74 which is adapted to receive a threaded shaft 103 which can be screwed into and out of the hub 74 by knob 104 to close off or open the flexible tubing 110 within body 70 — as required to control the flow of fluid therethrough in operation of the apparatus.

The nozzle 112 has a flared opening 90 at its discharge end which is positioned near the pads 60. The discharge opening 90 flares to a diameter of about 1.5 mm (.060 inch).

In operation of the invention, the device 10 is preferably calibrated by having the tubing 110 connected to source 120, such as water faucet or the like, to which it might be conveniently, but not necessarily, always connected and ready for use. The source 120 is turned on to maximum flow, and the valve knob 104 is manipulated to set the shaft 103 in the valve body 70 at the proper depth and contact with tubing 110 to provide the desired flow of water out of the tube 110. Uncontrolled high pressure flow might damage the eye, and it is undesirable to take time to set the flow at the moment when the device is needed for use.

In use, when it is desired to irrigate an eye in which an irritant has entered and whose lids are tightly closed because of the presence of the irritant, with water flowing out of the opening 90 in the tube 70, and with finger hole portions open and pads closed, the operator places the closed pads 60 on the two eyelids, and, by closing the finger hole portions 40 and 50, the pads are moved apart, the eye is exposed and fluid enters the eye at a favorable rate of flow. Additional ease of operation can be achieved in some cases by using one hand to assist in the elevation of the upper eyelid. Fingertips exert upward pressure in the region of the eyebrow/lid to assist the device in initially opening the eye. The fluid flow is continued for as long as required to cleanse and irrigate the eye of the irritating substance present.

The present medical consensus for the best immediate treatment of a caustic substance in the eye is by thoroughly irrigating the open eye continuously, but for short breaks, for at least 15 minutes.

It is noted that, when the blades are closed (Figs. 1 and 4), the raised studs or posts 61 and 62 engage the valve body 70 and properly center it so that the desired directional flow of fluid into the eye is achieved.

In a modification of the invention for professional use, the leading ends of the blades 20 and 30 which engage the eyelids are provided with occular speculum-type members 130 (Fig. 7) which engage and hold the eyelids open in well known fashion. Other end structures might also be used.

**Claims**

1. Eye irrigation apparatus (10) comprising a scissors-like mechanism comprising two blades (20, 30) each having a finger hole (40, 50) at one end and eyelid engaging means (60 or 130) at the other end, and which blades (20, 30) are pivotably connected (on 64), characterised in that there are provided a tubular valve body (70) disposed and supported between said blades (20, 30), the valve body being adapted to receive fluid from a source (120) via a tube (110) and being positioned so that fluid flowing in a stream out of said valve body (70) flows into and irrigates the eye when the apparatus is disposed in operative relation with the eye, and an adjustable valve means (74, 103, 104) in said valve body (70) for controlling the flow of fluid therethrough and settable over a range of settings to permit steady fluid flow therethrough at a selected velocity.

2. Apparatus as claimed in claim 1, wherein the eyelid engaging means comprises soft friction pads (60) which can engage and move the eyelids as they move apart.

3. Apparatus as claimed in claim 1 or 2, wherein the eyelid engaging means comprises optical speculum-type members (130).

4. Apparatus as claimed in any of claims 1 to 3, wherein the tube (110) is flexible and extends into said valve body (70).

5. Apparatus as claimed in any of claims 1 to 4, further including a raised stud (61, 62) on each blade (30, 20), the studs being positioned to hold said valve body (70) between them when the apparatus is in use whereby the valve body (70) is held and aimed properly so that fluid is directed at the eye as desired.

6. Apparatus as claimed in claim 5, in which said studs (61, 62) are on the facing inner surfaces of said blades (30, 20) rearwardly of the pivots of said blades (30, 20) and near the rear end of said valve body (70).

7. Apparatus as claimed in any of claims 1 to 6, in which said blades are arranged so that, when the finger hole (40, 50) ends are close together, the other ends (60 or 130) are open and spread apart, and vice versa.

8. Apparatus as claimed in claim 4, in which the adjustable valve means (74, 103, 104) is adapted to engage said flexible tube (110) within the valve body (70) to control the flow of fluid therethrough.

9. Apparatus as claimed in any of claims 1 to 8, in which said valve body (70) is pivotably coupled to said blades (20, 30) whereby both said blades (20, 30) can pivot with respect to said valve body.

**Patentansprüche**

1. Vorrichtung (10) zum Spülen des Auges mit einem scherenähnlichen Mechanismus mit zwei Flügelteilen (20, 30), wobei jedes mit einem Fingerloch (40, 50) an seinem einen Ende und Einrichtungen zur Erfassung des Augenlides (60 oder 130) am anderen Ende ausgestattet ist, wobei die Flügelteile (20, 30) untereinander verschwenkbar verbunden sind (bei 64), dadurch gekennzeichnet, daß ein röhrenförmiger Ventilkörper (70) zwischen diesen Flügelteilen (20, 30) angeordnet und gehalten ist, der Flüssigkeit von einer Flüssigkeitsquelle (120) über eine Röhre (110) erhalten kann, und der so angeordnet ist, daß die aus ihm ausströmende Flüssigkeit in das Auge einfließt und dieses benetzt, wenn die Vorrichtung in Berührung mit dem Auge gebracht wird, und daß einstellbare Ventilteile (74, 103, 104) in dem Ventilkörper (70) vorgesehen sind, um den Flüssigkeitsfluß durch das Ventil zu steuern, die über einen bestimmten Einstellbereich veränderbar sind, um einen kontinuierlichen Flüssigkeitsdurchfluß mit wählbarer Geschwindigkeit zu ermöglichen.

2. Vorrichtung nach Anspruch 1, wobei die Einrichtungen zur Erfassung des Augenlides weiche Kissen (60) beinhalten, die die Augenlider erfassen und bewegen können, wenn sie sich auseinander bewegen.

3. Vorrichtung nach Anspruch 1 oder 2, wobei die Einrichtungen zur Erfassung des Augenlides Teile (130) nach Art eines optischen Spiegels (Spekulum) enthalten.

4. Vorrichtung nach einem der Ansprüche 1—3, wobei die Röhre (110) flexibel ist und sich in den genannten Ventilkörper (70) erstreckt.

5. Vorrichtung nach einem der Ansprüche 1—4, wobei jedes Flügelteil einen abstehenden Steg (61, 62) beinhaltet, die so angeordnet sind, daß sie den Ventilkörper (70) zwischen sich halten, wenn die Vorrichtung in Gebrauch ist, wobei der Ventilkörper (70) derart gehalten und ausgerichtet wird, daß die Flüssigkeit dem Auge in der gewünschten Richtung zugeführt wird.

6. Vorrichtung nach Anspruch 5, wobei die Stege (61, 62) auf den gegenüberliegenden Innenseiten der Flügelteile (20, 30) angeordnet sind, hinter den Gelenken der Flügelteile (30, 20) und in der Nähe des rückwärtigen Endes des Ventilkörpers (70).

7. Vorrichtung nach einem der Ansprüche 1—6, wobei die Flügelteile (20, 30) so angeordnet sind, daß wenn die Enden mit den Fingerlöchern (40, 50) nahe beisammen sind, die anderen Enden (60 oder 130) offen oder auseinander gespreizt sind, und umgekehrt.

8. Vorrichtung nach Anspruch 4, wobei die einstellbaren Ventilteile (74, 103, 104) so angeordnet sind, um die flexible Röhre (110) im Ventilkörper (70) zu beaufschlagen, um den Flüssigkeitsdurchfluß zu steuern.

9. Vorrichtung nach einem der Ansprüche 1—8, wobei der Ventilkörper (70) mit den Flügelteilen (20, 30) drehbar verbunden ist, und wobei die Flügelteile (20, 30) relativ zu dem Ventilkörper (70) verschwenkbar sind.

**Revendications**

1. Appareil (10) pour l'irrigation des yeux, comportant un mécanisme semblable à des ciseaux, comprenant deux branches (20, 30) ayant chacune une ouverture (40, 50) pour un doigt à une extrémité et un moyen de déplacement de paupière (60 ou 130) à l'autre extrémité et ces branches (20, 30) étant liées à pivot (en 64), caractérisé en ce qu'il est prévu un corps de vanne tubulaire (70) disposé et porté entre lesdites branches (20, 30), le corps de vanne étant agencé pour recevoir du fluide à partir d'une source (120) par un tube (110) et étant positionné pour que du fluide sortant en un flux dudit corps de vanne (70) s'écoule dans l'oeil et l'irrigue quand l'appareil est disposé en liaison de fonctionnement avec l'oeil, et un moyen à vanne réglable (74, 103, 104) dans ledit corps de vanne (70) pour commander l'écoulement du fluide qui le traverse et pouvant être ajusté sur une échelle de réglages pour permettre un flux stabilisé du fluide à une vitesse choisie.

2. Appareil selon la revendication 1, dans lequel le moyen de déplacement de paupière comporte des tampons à frottement doux (60) qui peuvent attaquer et déplacer les paupières lorsqu'ils s'écartent.

3. Appareil selon la revendication 1 ou la revendication 2, dans lequel le moyen de déplacement de paupière comporte des éléments oculaires du type spéculum (130).

4. Appareil selon l'une quelconque des revendications 1 à 3, dans lequel le tube (110) est flexible et s'étend dans ledit corps de vanne (70).

5. Appareil selon l'une quelconque des revendications 1 à 4, comportant en outre un téton (61, 62) en saillie sur chaque branche (30, 20), les tétons étant positionnés pour maintenir ledit corps de vanne (70) entre eux quand l'appareil est utilisé, ce qui fait que le corps de vanne (70) est tenu et ajusté correctement pour que du fluide soit envoyé à l'oeil comme on le désire.

6. Appareil selon la revendication 5, dans lequel lesdits tétons (61, 62) sont sur les surfaces desdites branches (30, 20) vers l'intérieur, en arrière des pivots desdites branches (30, 20) et près de l'extrémité arrière dudit corps de vanne (70).

7. Appareil selon l'une quelconque des revendications 1 à 6, dans lequel lesdites branches sont agencées de manière que, quand les ouvertures de doigt (40, 50) sont rapprochées l'une de l'autre, les autres extrémités (60 ou 130) sont ouvertes et écartées, et vice versa.

8. Appareil selon la revendication 4, dans lequel le moyen à vanne réglable (74, 103, 104) est agencé pour agir sur ledit tube flexible (110) à l'intérieur du corps de vanne (70) pour commander l'écoulement du fluide qui le traverse.

9. Appareil selon l'une quelconque des revendications 1 à 8, dans lequel ledit corps de vanne (70) est lié à pivot auxdites branches (20, 30), lesdites deux branches (20, 30) pouvant ainsi pivoter par rapport audit corps de vanne.

Fig. 1

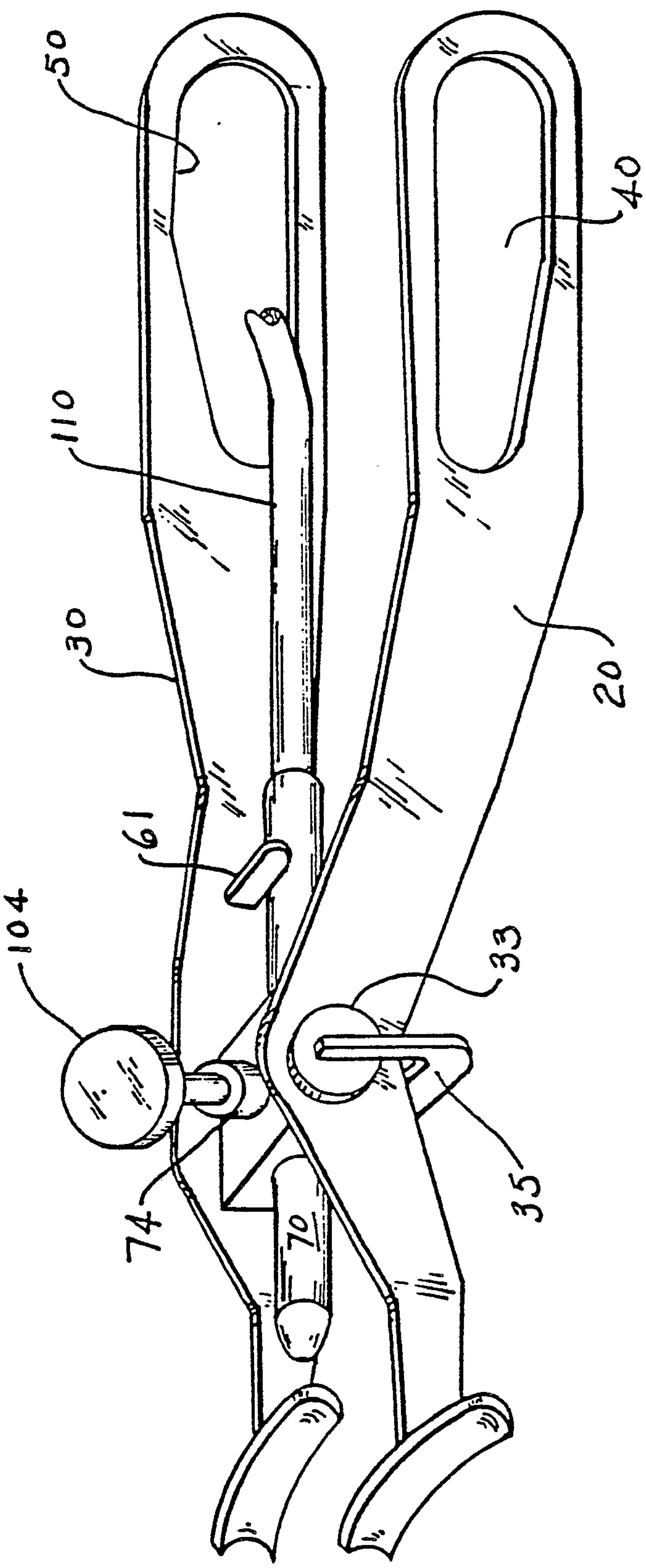

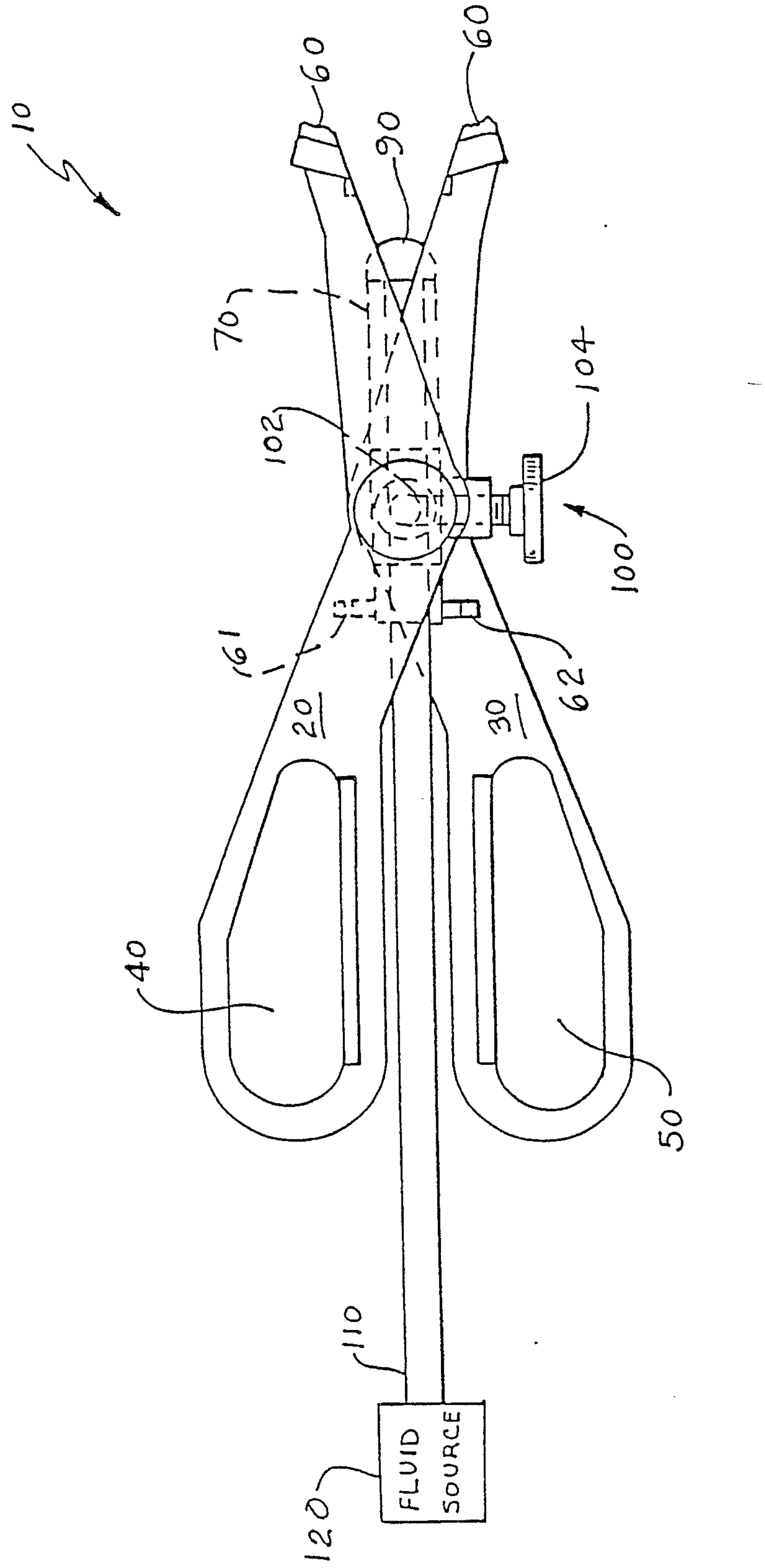


Fig. 2

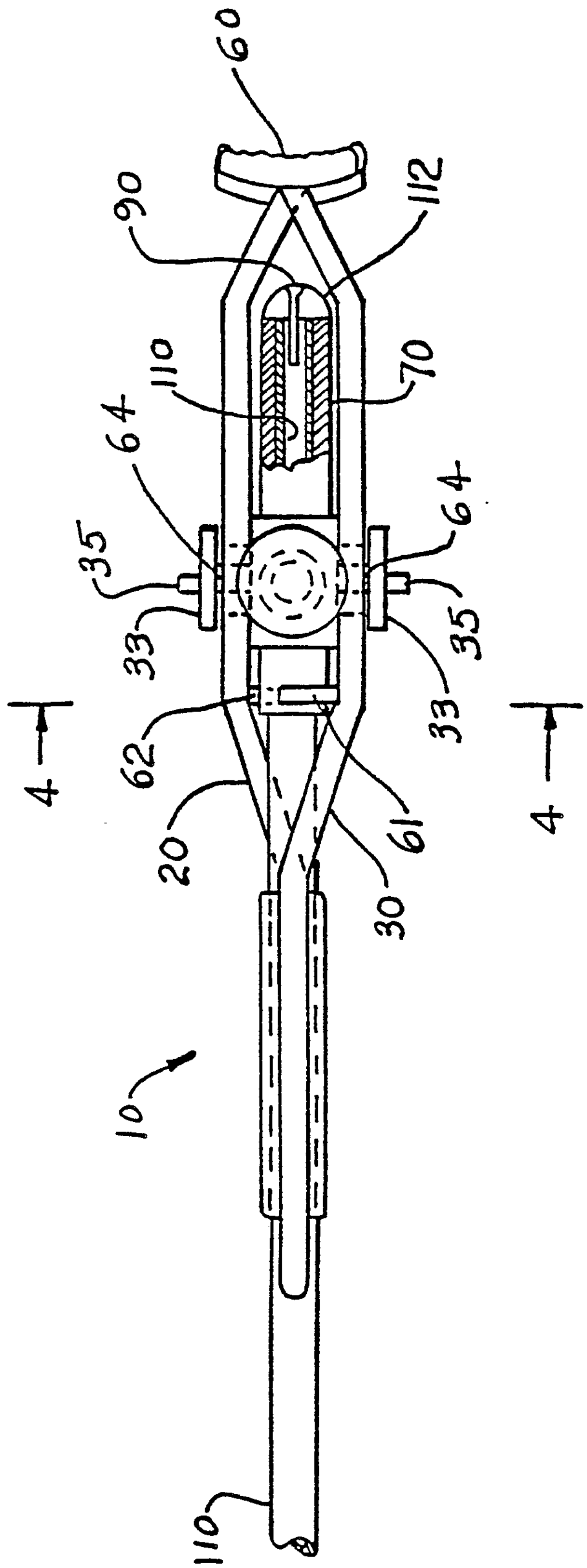


Fig. 3

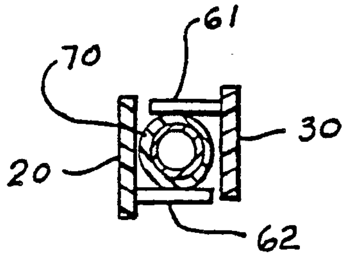


Fig. 4

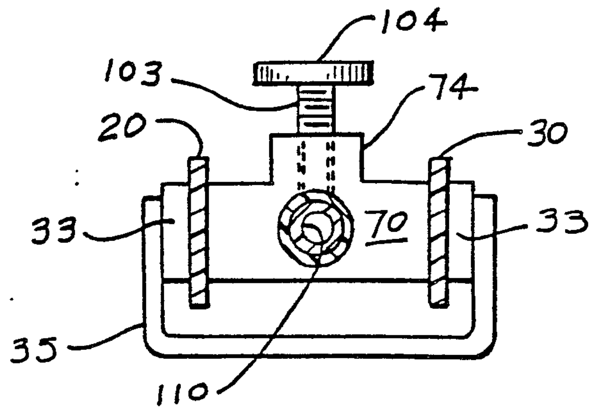


Fig. 5

Fig. 6

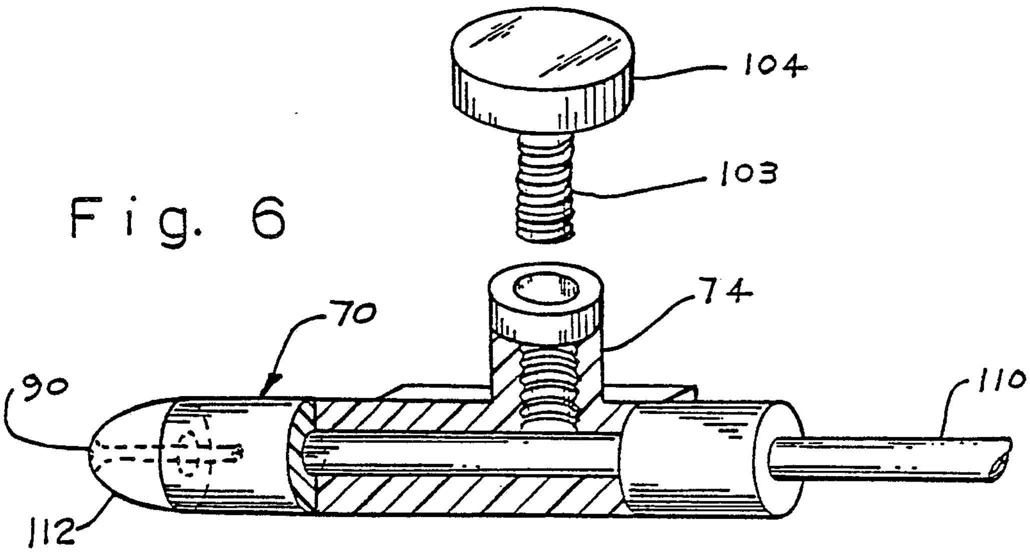


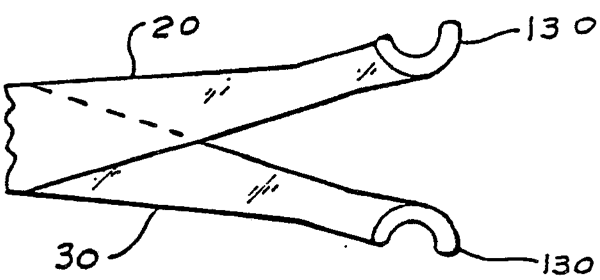


Fig. 7